# EUROPEAN PATENT SPECIFICATION

(11) **EP 3 714 765 B1**
(45) Date of publication and mention of the grant of the patent: **29.12.2021**
(21) Application number: 18849477.7
(22) Date of filing: 23.11.2018
(51) Int. Cl.: A61B 3/10, A61B 3/13

(54) **DEVICE AND METHOD FOR OBTAINING MECHANICAL, GEOMETRIC, AND DYNAMIC MEASUREMENTS OF OPTICAL SURFACES**
GERÄT UND VERFAHREN ZUM ERHALTEN VON MECHANISCHEN, GEOMETRISCHEN UND DYNAMISCHEN MESSUNGEN VON OPTISCHEN OBERFLÄCHEN
DISPOSITIF ET PROCÉDÉ D'OBTENTION DE MESURES MÉCANIQUES, GÉOMÉTRIQUES ET DYNAMIQUES DE SURFACES OPTIQUES

(30) Priority: 23.11.2017 ES 201731354
(43) Date of publication of application: 30.09.2020
(73) Proprietor: Fundacion Instituto De Investigacion Sanitaria Fundacion Jimenez Diaz, 28040 Madrid (ES); Universidad Politécnica De Madrid, 28040 Madrid (ES)
(72) Inventor: PÉREZ MERINO, Pablo, 28040 Madrid (ES); ALEJANDRE ALBA, Nicolás, E-28040 Madrid (ES); JIMÉNEZ-ALFARO MOROTE, Ignacio, 28040 Madrid (ES); FERNÁNDEZ LÓPEZ, Antonio, E-28040 Madrid (ES); GÜEMES GORDO, Jesús Alfredo, E-28040 Madrid (ES); LARRAÑAGA VALSERO, Beatriz María, E-28040 Madrid (ES)
(74) Representative: ABG Intellectual Property Law, S.L.
(86) International application number: PCT/ES2018/070757
(87) International publication number: WO 2019/102056

(56) References cited:
- EP-A1- 2 857 793
- EP-A1- 3 088 839
- WO-A2-2006/074310
- US-A1- 2015 018 661

## Description

### Field of the Art

The object of this invention relates to a new device for obtaining mechanical, geometric, and dynamic measurements of ocular surfaces, preferably the cornea, or optical surfaces (such as lenses, ophthalmic lenses, contact lenses, intraocular lenses, or self-adjusting lenses).

Three-dimensional measurements of the mechanics of the cornea, three-dimensional dynamic measurements of the cornea-tear film, geometric measurements of the cornea and sclera, precise measurements of intraocular pressure, and finally, geometric and mechanical measurements of ophthalmic elements, optical elements, and electro-optical devices, are obtained by means of the device of the invention.

Said measurements are obtained for the purpose of: (a) determining the threshold value in subjects without an ocular pathology; (b) evaluating subjects with refractive errors and their treatment with refractive surgery, contact lenses, and intraocular lenses; (c) three-dimensionally characterizing the surface shape and the mechanics of optical elements (ophthalmic lenses, contact lenses, intraocular lenses, etc.) and ophthalmic, optical, and electro-optical devices (self-adjusting lenses, integrated optics, etc.); and (d) improving predictability in the diagnosis of at least the following ocular pathologies and their associated treatments:
i. pathologies weakening the corneal structure such as corneal ectasia and keratoconus (treatments: intracorneal rings, corneal cross-linking, corneal transplantation);
ii. glaucoma (treatments: topical medication which reduces intraocular pressure and surgery); and
iii. dry eye (treatment: artificial tears, eye drops, plugs).

The present invention is applicable in the field of medicine, and more specifically in the field of ophthalmology.

EP-A-3 088 839 discloses a device for obtaining mechanical, geometric, and dynamic measurements of optical surfaces comprising a high-speed stereoscopic imaging system, a first projector configured for projecting a random infrared illumination pattern onto the entire surface of an optical region, a second projector configured for projecting an illumination pattern projected on the central area of the optical region, a processor configured for calibrating the high-speed stereoscopic imaging system, calculating the position of the high-speed stereoscopic imaging system in space, establishing depth ratios in the high-speed stereoscopic imaging system, and determining the position of the optical region in space and the depth ratio of the optical region with respect to the high-speed stereoscopic imaging system, wherein the high-speed stereoscopic imaging system is configured for obtaining at least one image of the random infrared illumination pattern projected onto the entire surface of the optical region, as well as of the illumination pattern projected onto the central area of the optical region, wherein the processor is configured for generating a final image with three-dimensional information about the entire extension of the optical region using a superposition, by means of digital image correlation, of the images obtained by means of the high-speed stereoscopic imaging system.

### Background of the Invention

The cornea is the thick transparent part of the sclerocorneal layer surrounding the eye, where the cornea makes up one-sixth of said layer, is located in the front portion thereof, and covered by the tear film. The most significant characteristics of the cornea are its transparency, refraction, and structural support function providing, together with the sclera (the white part of the eye), protection to the eyeball.

Focusing on the cornea, it can be said that the cornea is, according to these characteristics, a (necessarily transparent and protective) window and a lens (which provides 2/3 of the refractive error of the eye). However, in certain cases, this tissue may become afflicted with pathologies such as keratoconus or corneal ectasias and it may be the object of traumas, with the weakening of the structural integrity thereof.

On the other hand, since it is the main refractive element of the eye, the cornea has become the preferred site for the performance of various refractive surgery techniques that seek to reduce reliance on glasses, removing the tissue and weakening the corneal structure to a certain degree. Furthermore, the corneal surface is the surface where contact lenses are fitted.

Furthermore, the cornea is an ocular tissue that is highly linked to ocular fluids. In fact, its cellular framework allows internal fluid passage from the aqueous humor and external fluid passage from the tear film, maintaining the metabolic mechanism of the cornea. Any condition adversely affecting the stability and function of the aqueous humor and the tear film results in the onset of glaucoma and dry eye, respectively.

Finally, the cornea is key to the diagnosis and treatment of glaucoma because its characteristics influence both the intraocular pressure (IOP) measurement and the analysis of the progression of the disease, given that the evaluation techniques take the IOP measurement by determining the resistance of the cornea to an applied load. IOP is the pressure exerted by intraocular liquids against the wall of the eye, which is necessary to maintain the structural integrity of the eyeball.

The cornea has three main functions: light refraction, light transmission, and structural support and protection for the eyeball. Any change which alters corneal morphology will alter visual function. For this reason, the three-dimensional understanding of its shape, dynamics, and mechanical response to external agents (such as a disease, injury, surgery, or tear film instability) or internal agents (an increased IOP), is of great clinical importance.

Until a relatively short time ago, it was only possible to take merely geometric measurements of the cornea with a single acquisition, such as the radius of curvature, asphericity, thickness, and topography; however, due to the enormous interest in determining the three-dimensional dynamic and mechanical characteristics of the cornea for predicting the surgical response, assessing the risk of ectasia, analyzing the dynamics of the tear film in the entire extension thereof, and accurately measuring IOP (by including three-dimensional mechanical and geometric parameters), different techniques have been developed in recent years.

The term "mechanics" of the cornea (or "biomechanics" of the cornea) is strongly linked to the concept of viscoelasticity which, based on stress-strain ratios, allows classifying materials as elastic solids, non-viscous fluids, and viscous fluids. Elasticity is the study of the relationships existing between applied forces and the strains they cause. This concept can be defined as the resistance of a material to strain when a force is applied, and this term is quantified by the modulus of elasticity or Young's modulus. A material having high elasticity has greater resistance to strain. In an elastic material, once the applied force is eliminated, the material returns to its original state.

In the cornea, the load is applied internally by the IOP which generates stress in the cornea (the external atmospheric pressure maintains an optimum IOP level and the corneal structure in dynamic equilibrium). The thickness of the cornea is not uniform along its surface, which means that the distribution of stress will not be uniform either; the regions of smaller thickness will therefore have a higher stress than thicker areas. Nevertheless, the elastic response of the cornea has no linearity, having a lower modulus of elasticity with low stresses and a higher modulus of elasticity with high stresses, so when IOP increases, the resistance of the cornea to strain also increases. The elastic response only depends on the magnitude of the applied force, not on time.

Viscoelasticity is another property of materials which indeed depends on time, and the resistance to an applied force will also depend on the speed at which the force is applied (it describes the speed at which the material flows).

The cornea is formed by several layers composed of various cells, collagen fibers, and extracellular matrix, forming substructures which are characterized by a specific response to the application of a force. The stress-strain ratio depends on the relative contributions of the supporting constituents. These supporting elements have different behaviors, with some being more elastic and others having a more viscous behavior, and when attached together, they provide the structure with a viscoelastic-type mixed behavior. The response of a viscoelastic material is a function of the speed at which the force is applied and the time in which the force is applied, characterized by a progressive elastic strain and recovery starting from its initial shape.

Some of the mechanical parameters described based on *ex vivo* experiments performed on corneas for the analysis of the corneal tissue are: elastic modulus or Young's modulus (E), rigidity, viscoelastic parameters (E(t), T, P), creep or strain and hysteresis. Young's modulus and the coefficient of rigidity are two physical parameters expressing elastic properties of the eye. An elastic material recovers its original configuration upon removal of the applied force. In contrast, viscoelastic materials return to their original configuration, but not in the same way as when the force is applied thereto, and it depends on the magnitude of said stress. This property is known as hysteresis. Creep or strain describes the resistance of the cornea to an associated load.

The mechanical properties of the cornea and its constitutive materials are important for analyzing the connection between morphology and mechanical behavior. Imaging technique based on corneal topography and Scheimpflug image are the clinical standards in three-dimensional corneal surface analysis.

However, there is no instrument in clinical practice which provides quantitative three-dimensional information about the mechanical properties of the corneal structure. Furthermore, there is no commercial intraocular pressure measurement system (a critical measurement for glaucoma screening and treatment) which considers in its result the mechanical properties of the cornea which constitute the critical parameter for a precise analysis of intraocular pressure (IOP).

The *in vivo* evaluation of the dynamic and mechanical surface properties of the cornea through non-invasive procedures is an emerging field. Two devices have been marketed in recent years: ORA (Ocular Response Analyzer, Reichert Ophthalmic Instruments Inc, Depew, NY) and CORVIS ST (OCULUS Optikgeräte GmbH, Wetzlar, Germany), and several experimental systems based on optical coherence tomography (OCT) have been developed.

Both the commercial systems and the experimental developments combine the acquisition of an image and mechanical stimulation of the corneal structure (strain).

Finally, there are also different publications which have experimentally been demonstrated to obtain, with Brillouin microscopy, data of the so-called "Brillouin modulus" of the cornea. The Brillouin technique arises from the interaction of light and sound wave with tissue. However, Brillouin had not been fully examined in ophthalmology because it requires very long acquisition times (which are rather impractical for the *in vivo* screening of a patient's cornea) and does not provide intrinsic absolute mechanical parameters (the relationship between the Brillouin modulus and Young's modulus as well as their comparison are still under study). It must be taken into account that, besides the discomfort of being subjected to a lengthy screening, the human eye experiences spontaneous movements *in vivo* (associated with the continuous scanning the eyes perform (saccadic movements) and other movements associated with heartbeat and breathing), which will logically affect the acquisition of measurements, particularly if the screening is prolonged in time.

The mechanical stimulation in commercial ORA and CORVIS ST systems, as well as in some devices developed based on optical coherence tomography (OCT), is performed by means of a strong air puff directed to the central area of the cornea producing strain in the adjacent internal structures of the eye. Another form of mechanical stimulation is acoustic stimulation, in which the vibration response to resonance frequencies is analyzed.

The two marketed devices (ORA and CORVIS ST) represent the first combined systems for measuring the mechanical properties of the cornea and intraocular pressure.

ORA is a technique which measures changes in the reflectivity of the corneal surface, while a strong, sustained air puff, which centrally strikes the cornea, maintains a strain on said cornea; a photodetector picks up two bidirectional axial applanation instants associated with changes in external and internal pressure, offering the values of resistance of the cornea and hysteresis (mechanical parameters of the cornea) and the IOP measurement.

The ORA technique has the following drawbacks:
- it is one-dimensional in nature (it measures displacement in the direction of application of the strong air puff);
- it does not obtain an image of the strain of the cornea, but rather stationary records of two isolated strain instants of the cornea;
- it performs a very indirect estimate of the parameters related to corneal hysteresis and there is an enormous variability in this measurement given that it is taken in one dimension and in an isolated instant and there is a need to consider that the corneal structure is three-dimensional.

In turn, CORVIS ST also uses the incidence of the strong, sustained air puff in the central part of the cornea and measures the strain thereof using Scheimpflug images (Scheimpflug images offer a greater depth of focus than conventional images, when the prolongation of the object plane, lens, and sensor coincide at a common point) in a single meridian, offering a series of spatial and temporal strain parameters directly related to the mechanics of the cornea. However, it is a technique that only uses mechanical information about the cornea in a single meridian (two dimensions).

Furthermore, it must be pointed out that both ORA and CORVIS ST present an enormous variability in the measurement of intraocular pressure in comparison with the existing techniques for measuring IOP.

In addition to the foregoing, it is reiterated that the cornea is a complex anisotropic tissue, i.e., it shows different physical properties, such as elasticity, viscosity, or a combination of both, when stress is applied in different directions because the different microstructures of the cornea have different mechanical properties. These properties are related to the surface shape of the cornea and are fundamentally determined by the interaction of different materials such as collagen and extracellular matrix. However, the cornea is furthermore a highly heterogeneous structure from the center to the periphery, from the anterior surface to the posterior surface, and in its rotational dimensions, and these properties are in turn not constant because they change with age and with the pathology of the cornea. For that reason, one-dimensional approaches (ORA) or single-meridian approaches (CORVIS ST) are not precise enough for the mechanical analysis of the cornea.

Therefore, none of the marketed systems (ORA and CORVIS ST) offers three-dimensional mechanical, geometric, and dynamic information about the cornea which is required for reducing the variability associated with one- or two-dimensional techniques. This therefore leads to the need to propose an alternative which allows precisely extracting information about the corneal surface in a dynamic manner, the mechanical properties of the cornea in the entire extension thereof, and finally determining intraocular pressure. The three-dimensional surface, dynamic, and mechanical information can therefore be used for precisely reconstructing the intraocular pressure and the dynamics of the tear film in any type of ocular condition affecting the cornea (keratoconus, refractive surgery, corneal transplantation, dry eye, glaucoma).

Furthermore, up until now, the measurement systems existing on the market and many of the experimental developments based on optical coherence tomography (OCT) use a strong air puff which directly strikes the central part of the cornea to cause considerable mechanical strain of the tissue, where it constitutes a bothersome and uncomfortable (and, in practice, unfeasible) option for the patient and causes a sudden palpebral reflex and a displacement in the adjacent and internal structures of the eye.

OCT is a complex, high-cost imaging technique that requires special illumination sources (such as superluminescent diode or swept laser sources, for example), recording systems such as line scan cameras (spectral domain configuration) or photodiode (swept laser configuration), and an optical scanner which requires a sequential scanning over time for the three-dimensional reconstruction of the interference signal on sample surfaces.

The OCT technique is based on gradually performing a line/meridian scan of the surface of the eye and does not detect the entire surface in 3D in a single acquisition, a circumstance which (together with the use in some proposals of a strong, sustained air puff straining the eye and directly striking the central area of the cornea) causes this approach to have some limitations.

Furthermore, it is important to point out that OCT is not a technique which directly records images given that they are acquired by signal processing algorithms which calculate the location where the interference occurs. Once the signal from the scan is detected, the system must be calibrated to compensate for the intrinsic distortions of the system before proceeding to the three-dimensional reconstruction. Therefore, the processing of information with this technique is not immediate. Furthermore, as mentioned in the preceding paragraph, the temporal nature of the scan associated with the scanner also limits the immediacy in the acquisition of this technique, with it being sensitive to ocular movements.

Therefore, the search for a less complex, low-cost measurement technique which is capable of three-dimensionally quantifying, in a precise and immediate manner, the surface and mechanical parameters of the cornea, and which eliminates the invasive nature of the strong air puff striking a central area of several square millimeters in the cornea, would constitute an ideal and isolated approach to problems associated with fixation, involuntary axial movements associated with protective reflex reaction, and excessive blinking.

Document US 7 871 378 B1 is known in the state of the art and discloses an optical device based on the Scheimpflug principle, where Scheimpflug images of the cornea are obtained in a horizontal meridian (two dimensions) while the cornea is strained with a central air puff.

Documents US 2010/238408 A1 and ES 2571209 B1 are also known and they propose two topography systems capable of obtaining three-dimensional geometric information about the corneal surface in combination with a sustained central air puff.

Document US 2010/0238408 A1 relates to a dynamic rasterstereography system in which the corneal surface is stained and a linear grid (regular and non-random pattern) is then projected on the cornea.

Document ES 2571209 B1 relates to a plenoptic topography system combining an array of microlenses and a high-speed sensor.

Both systems are intended for a three-dimensional analysis with an invasive approach of the sustained air puff in the central area of the cornea.

Besides contemplating a bothersome mechanical stimulation with an air puff that strikes the central area of several square millimeters, document US 2010/0238408 A1 relates to a technique that has a limited spatiotemporal resolution and is based on a single conventional camera (which would limit its three-dimensional precision), a feature which would limit a precise analysis in irregular corneas like in the case of those affected by keratoconus; in turn document ES 2571209 B1 relates to a technique which possesses certain complexity in terms of hardware, does not obtain the three-dimensional shape of the cornea, and has a limited temporal resolution, likewise limiting a precise mechanical quantification in irregular corneas (for example, keratoconus or corneal ectasias).

The document by Twa et al., Biomed Opt Express 2014: "Spatial characterization of corneal biomechanical properties with optical coherence elastography after UV cross-linking*"* is also known, and it describes the experimental use of an air puff focused on the scleral region combined with the optical coherence tomography (OCT) imaging system; this development allowed for a non-invasive and therefore less bothersome corneal mechanical stimulation method. Up until now, its application has only been demonstrated in corneas *ex vivo* (eyes of sacrificed animals or human donors). *Ex vivo* corneal analyses have a limited scope as they do not take into account the involuntary movements produced in the eye of a living patient. Therefore, the *ex vivo* analysis results cannot be readily extrapolated to the eyes of living patients.

Likewise, document US 2013/329953 A1 uses digital image correlation (DIC) in objects, where an external vibration is applied and a speckle pattern applied in a completely invasive manner by means of adhesives or stains on the surface of different materials is detected.

Document CN 103674829 A uses the digital image correlation (DIC) technique for measuring the mechanical parameters of corneas *ex vivo,* where a speckle pattern is applied in an invasive manner by means of an adhesive or a stain painted on the actual surface of the corneal tissue and the eye is inflated by inserting a needle or another element through the optic nerve for the purpose of determining the biomechanics in donor eyes *ex vivo.*

The digital image correlation technique (known with the acronym DIC) is an optical technique which allows measuring strains of the object being tested by means of analyzing the vectors of displacement from an initial state to the strained state, where it constitutes an analysis technique with more affordable hardware, with images of the surface to be measured being obtained directly (not by reconstruction like in the case of OCT).

The DIC technique can be implemented in two dimensions (analyzing displacements in a single plane using a single camera: 2D DIC), or in three dimensions (analyzing displacements in a plane and outside a plane, using two cameras, for example: 3D DIC) .

The DIC technique has been used in the state of the art for evaluating the mechanical response to tissue strain *ex vivo,* (and it has not been contemplated for ocular tissues *in vivo* due to its invasive characteristic, as it is not possible to treat the corneal surface of a patient with a staining pattern or an adhesive (which is valid for material samples but not for a living tissue, and even less so for a tissue covered with a constantly renewed viscous fluid like the tear film), or to cause the eye inflation.

The biomechanical analysis parameters of the described commercial and experimental techniques are as follows:
- ORA: offering a direct one-dimensional measurement (in the direction of application of the strong air puff) of the following coefficients: corneal resistance factor (CRF) and corneal hysteresis (CH);
- CORVIS ST: offering a direct two-dimensional measurement (along a meridian of the surface of the eye) of the maximum strain amplitude, applanation length, maximum concavity time, temporal symmetry factor, and corneal velocity.
- OCT: offering *ex vivo* elastic parameters (Young's modulus) and viscoelastic parameters (T and P constants), and with vibration, strain on the surface has been described as wave propagation, analyzing its speed and amplitude. The OCT technique uses an optical scanner for sweeping the corneal surface point-to-point and a subsequent reconstruction for obtaining three-dimensional data.

According to the foregoing, a three-dimensional dynamic measurement technique for measuring the mechanical characteristics of corneas *in vivo* in patients is not available today (all the published results have been described in corneas *ex vivo).*

The applicant is unaware of any document of the state of the art which covers the demands for measuring the geometric, mechanical, and/or dynamic properties of the cornea in a three-dimensional manner with a single non-invasive system. Furthermore, there is a need to incorporate three-dimensional geometric parameters of the corneal surface and three-dimensional mechanical parameters for determining intraocular pressure with greater precision.

### Description of the Invention

In order to solve the drawbacks and bridge the gaps mentioned in the state of the art, the following device for obtaining mechanical, geometric, and dynamic measurements of optical surfaces, of the eye, or of other optical elements, and the method of use thereof, is described.

The device for obtaining mechanical, geometric, and dynamic measurements of optical surfaces object of the present invention includes:
- a high-speed stereoscopic imaging system;
- an isolated stimulus generator configured for directing an spatially discrete stimulus to a first optical region Z P and to a second optical region;
- a first projector configured for projecting a random infrared illumination pattern onto the entire surface of the second optical region;
- a second projector configured for projecting a slit illumination pattern projected on the central area of the second optical region;
- a processor configured for: calibrating the high-speed stereoscopic imaging system; calculating the position of the high-speed stereoscopic imaging system in space; establishing depth ratios in the high-speed stereoscopic imaging system; and determining the position of the second optical region in space and the depth ratio of the second optical region with respect to the high-speed stereoscopic imaging system.

The high-speed stereoscopic imaging system is configured for obtaining at least one image of the random infrared illumination pattern projected onto the entire surface of the second optical region, as well as of the slotted illumination pattern projected onto the central area of the second optical region, all this without the application of the spatially discrete stimulus.

The high-speed stereoscopic imaging system is also configured for obtaining images of the random infrared illumination pattern and of the slotted illumination pattern during and after the application of the isolated stimulus which strikes the first optical region and/or the second optical region and propagates over the surface of the second optical region.

The processor is configured for generating a final image with three-dimensional information about the entire extension of the second optical region, using a superposition, by means of digital image correlation (DIC), of the images obtained by means of the high-speed stereoscopic imaging system.

According to a possible aspect of the invention, the first optical region is the sclera of an eye and the second optical region is the cornea of the eye.

Alternatively, according to another possible aspect of the invention, the first optical region is a perimetral area of an optical surface (an optical or electro-optical element or device) and the second optical region is a central area of said optical surface, where said optical surface can be: an optical lens, an ophthalmic lens, a contact lens, a intraocular lens, or a self-adjusting lens.

Additionally, according to a possible embodiment of the device of the invention, the first projector comprises a plurality of infrared light LEDs and is configured for generating the random infrared illumination pattern.

Also according to a possible embodiment of the device of the invention, the second projector comprises a white light source for generating the slotted illumination pattern.

Preferably, the first projector is configured so that each beam of the random infrared illumination pattern strikes the surface of the second optical region and is reflected such that each beam reflected on the surface of the second optical region is directed to an image plane where the high-speed stereoscopic imaging system obtaining a point distribution of the random infrared illumination pattern is located.

According to a preferred embodiment of the device of the invention, said device comprises a synchronization unit configured for synchronizing, in real time, the images obtained by means of the high-speed stereoscopic imaging system with the spatially discrete stimulus focused on the first optical region and/or on the second optical region, as well as with the random infrared illumination pattern and with the slotted illumination pattern.

Preferably, the device object of the present invention includes a storage module for storing the images obtained by means of the high-speed stereoscopic imaging system, where said storage module is connected to the processor.

According to a possible embodiment of the device object of the present invention, the device includes an additional fixation channel configured for controlling fixation of a user/patient. This feature is particularly useful in that aspect of the invention according to which the first optical region is the sclera of the eye and the second optical region is the cornea of the eye.

According to a possible embodiment of the device object of the present invention, the high-speed stereoscopic imaging system comprises a high-speed stereoscopic camera.

According to another possible embodiment of the device object of the present invention, the high-speed stereoscopic imaging system comprises at least two high-speed cameras.

According to a possible embodiment of the device object of the present invention, the isolated stimulus generator comprises a micro-air pulse generator configured for directing a micro-air pulse to the first optical region and/or to the second optical region.

The present invention also relates to a method for obtaining mechanical, geometric, and dynamic measurements of optical surfaces.

The method for obtaining mechanical, geometric, and dynamic measurements of optical surfaces comprises:
- projecting, by means of a first infrared light projector, a random infrared illumination pattern onto the entire surface of a second optical region;
- projecting, by means of a second projector, a slotted illumination pattern on the central area of the second optical region;
- acquiring, by means of a high-speed stereoscopic imaging system, at least one resting-state image of the reflection of the illumination patterns on the second optical region;
- recording, by means of the high-speed stereoscopic imaging system, at least one resting-state image of the morphological patterns of a first optical region and of the second optical region;
- applying, by means of a spatially discrete stimulus generator, a spatially discrete stimulus focused on the first optical region and on the second optical region;
- acquiring, by means of the high-speed stereoscopic imaging system, a plurality of high-speed images of the reflection on the second optical region of the projected illumination patterns;
- again recording, by means of the high-speed stereoscopic imaging system, a plurality of high-speed images of the morphological patterns of the first optical region and of the second optical region;
- removing the spatially discrete stimulus that has been applied;
- acquiring, by means of the high-speed stereoscopic imaging system, a plurality of high-speed images of the reflection on the second optical region of the projected illumination patterns without incidence of the spatially discrete stimulus;
- again recording, by means of the high-speed stereoscopic imaging system, a plurality of high-speed images of the morphological patterns of the first optical region and of the second optical region; and
- measuring mechanical, geometric, and dynamic parameters of the second optical region in the recovery thereof from the removal of the spatially discrete stimulus to the state prior to the application of the spatially discrete stimulus.

According to one aspect of the method of the invention, the first optical region is the sclera of an eye and the second optical region is the cornea of the eye.

Alternatively, according to another possible aspect of the method of the invention, the first optical region is a perimetral area of an optical surface and the second optical region is a central area of said optical surface, where said optical surface can be: an optical lens, an ophthalmic lens, a contact lens, an intraocular lens, or a self-adjusting lens.

In the aspect of the invention according to which the first optical region is the sclera of an eye and the second optical region is the cornea of the eye, the morphological patterns of the first optical region and the second optical region can be the edges and morphological patterns of the eye such as the edge of the pupil, the white of the eye, and vessels of the eye. This allows the recording of the morphological patterns present in the image to be used as an additional "pattern" characteristic of each eye.

According to a possible embodiment of the invention, the method comprises projecting the random infrared illumination pattern by means of a plurality of infrared light LEDs configured for generating the random infrared illumination pattern.

Likewise, according to a possible embodiment of the invention, the method comprises projecting the slotted illumination pattern by means of a white light source configured for generating the slotted illumination pattern.

Preferably, the method of the invention comprises projecting the random infrared illumination pattern such that each beam of the random infrared illumination pattern strikes the surface of the second optical region and is reflected such that each beam reflected on the surface of the second optical region is directed to an image plane where the high-speed stereoscopic imaging system obtaining a point distribution of the random infrared illumination pattern is located.

According to a possible embodiment, the method comprises applying the spatially discrete stimulus in the form of a micro-air pulse, for which the isolated stimulus generator comprises a micro-air pulse generator configured for directing a micro-air pulse to the first optical region and/or to the second optical region.

In the aspect of the invention according to which the first optical region is the sclera of an eye and the second optical region is the cornea of the eye, the device of the invention can be used as a dynamic corneal topography system by means of acquiring images without incidence of the spatially discrete stimulus.

The device of the invention can be used for the three-dimensional analysis of the geometric, mechanical, and dynamic properties of the cornea.

The device of the invention can be used for the analysis of intraocular pressure.

In the aspect of the invention according to which the first optical region is a perimetral area of an optical surface and the second optical region is a central area of said optical surface, where said optical surface can be: an optical lens, an ophthalmic lens, a contact lens, an intraocular lens, or a self-adjusting lens, the device of the invention can be used for the three-dimensional analysis of the shape and mechanics of said optical surface.

The device of the invention can be used for the evaluation, classification, and follow-up of people without ocular pathologies, for determining the threshold value indicative of the onset of at least the following ocular pathologies:
i. pathologies weakening the corneal structure such as corneal ectasia and keratoconus;
ii. glaucoma; and
iii. dry eye;
thereby improving predictability in the diagnosis of said ocular pathologies and enabling the determination of a suitable treatment for said pathologies.

Likewise, the device of the invention can be used for the evaluation, classification and follow-up of people with refractive errors (myopia, hypermetropia, etc.), for determining, based on said errors, a suitable treatment selected from one or more of at least: treatment with refractive surgery, contact lenses, and intraocular lenses.

In summary, the present invention describes an innovative device for the three-dimensional dynamic measurement of the cornea with a discrete and non-invasive stimulation system, by means of a novel approach of projecting the random illumination pattern associated with the DIC technique.

The device for obtaining mechanical, geometric, and dynamic measurements of optical surfaces, object of the present invention allows analyzing:
- the corneal surface in the entire extension thereof in a dynamic manner (without the application of a sustained mechanical stimulation over time);
- the mechanics of the cornea in three dimensions (by analyzing the variations of the cornea from the resting state to the strained state, and its recovery);
- intraocular pressure considering the surface and mechanical parameters of the cornea; and
- the surface and mechanics of optical and electro-optical elements.

The technique object of the present invention has at least the following advantages:
- 3D analysis of the entire area vs. a single section (2D) with CORVIS ST or axial displacement (1D) with ORA;
- it is a non-invasive technique (isolated stimulation/discrete micro-air pulse focused on the scleral and/or corneal region of the eye and propagated over the cornea) vs. methods that are invasive for the cornea (heavy air puff impacting the center of the cornea);

- high resolution (displacement analysis having a precision in the order of microns);
- high strain interval;
- field of vision: complete;
- three-dimensional stress-strain map;
- three-dimensional information processing that is immediate (single three-dimensional image acquisition in each moment associated with the load) and less complex (without requiring image reconstruction) vs. OCT (limited in temporal resolution for three-dimensional reconstruction as it depends on the time associated with the scan of the scanner); and
- capacity for dynamically evaluating the corneal surface (in a relaxed state).

The field of application of the present invention in the area of ophthalmology is very extensive and, among other applications, includes:
- identifying the biomechanical properties of the cornea (elastic modulus or Young's modulus, viscoelastic parameters, creep or strain, relaxation, rigidity, and hysteresis), obtaining an real measurement of intraocular pressure both in normal subjects and in patients with corneal alterations (such as corneal ectasias, for example) and patients with glaucoma. Furthermore, the dynamic measurements of the tear film are fundamental in the diagnosis and follow-up of patients with ocular surface alterations, particularly in dry eye syndrome and in both anterior and posterior blepharitis.
- diagnosing and stratifying the degree of development of ocular diseases presenting with alterations in the geometric, biomechanical, and/or dynamic properties of the cornea. In fact, with respect to other commercial equipment, the present invention allows early diagnosis of those ocular pathologies in subclinical stages by increasing the sensitivity and specificity in diagnosis as it combines three-dimensional information about the shape, biomechanics, and dynamics of the cornea in a single system. Risk factors in corneal alterations (keratoconus, corneal ectasias), dry eye, and glaucoma are thereby identified. Likewise, the prediction, safety, and efficacy of the refractive results in corneal refractive surgery, in which it is fundamental to properly screen the patients to reduce the risk of ectasia after the intervention, are improved.
- depending on the given diagnosis of a specific pathology, indicating the supportive treatment to be provided in the treatment of said disease. This will thereby allow the determination of the biomechanical effect of corneal treatments with contact lenses, intracorneal rings, cross-linking, and corneal transplantation, information that is lacking with the methods available up until now, the evaluation of the effect of treatments associated with dry eye (for example, artificial tears), in which there are virtually no target biomarkers available today which allow said evaluation, and the objective evaluation of topical and surgical treatments associated with glaucoma.

Furthermore, the characterization of optical and electro-optical elements and devices of ophthalmic application can be mentioned among other applications.

A table comparing the technologies described in the state of the art and the instrument/device object of the present invention is included below.

**Table 1. Progress of the technique object of the present invention with respect to the state of the art**

| | Clinical | | Experimental | Invention |
|---|---|---|---|---|
| | ORA | CORVIS ST | OCT | |
| 3D corneal biomechanics + corneal shape | X | X | √ (corneal shape: distortion correction algorithms) | √ |
| Biomechanics evaluation | Rigidity, hysteresis | Applanation length and speed, concavity | Elastic modulus, creep, relaxation, rigidity, hysteresis | Elastic modulus, creep, relaxation, rigidity, hysteresis, viscoelastic modulus |
| Actual intraocular pressure | X | X | √ Corrected with FEM (3D cornea) | √ Corrected with FEM (3D cornea) |
| Precision of displacement | Limited (2D) | Limited (2D) | High resolution (OCT < 10 µm) | High resolution (DIC < 10 µm) |
| High speed + 3D image | X | X | √ | √ |
| Strain through corneal thickness | √ (limited to 2D) | √ (limited to 2D) | √ | √ (limited to 2D) |
| Procedure that is invasive to the cornea | X (invasive) | X (invasive) | X (invasive: heavy central air puff) | √ (minimally invasive) |
| Profitability /cost of the technique | low | low | low | high |
| Tear film dynamics in 3D | X | X | X | √ |
| Image processing and simple 3D reconstruction | X | X | X | √ |

### Brief Description of the Drawings

Figure 1 shows a schematic depiction of an embodiment of the device of the invention for the three-dimensional analysis of the mechanical properties of the cornea.
Figure 2 shows the design of the random infrared illumination pattern.
Figure 3 shows a diagram illustrating corneal stimulation by means of a micro-air pulse focused on the sclera.

### Preferred Embodiment of the Invention

The device for obtaining mechanical, geometric, and dynamic measurements of optical surfaces object of the invention comprises:
- a high-speed stereoscopic imaging system (1);
- a spatially discrete stimulus generator (2), configured for generating a spatially discrete stimulus (8) in a first optical region (5), which propagates through a second optical region (6);
- a first projector (3) with LEDs of different sizes/diameters which projects a random infrared illumination pattern projected onto the entire surface of the second optical region (6); and
- a second white light projector (4) which projects a slotted illumination pattern projected on the central area of the second optical region (6).

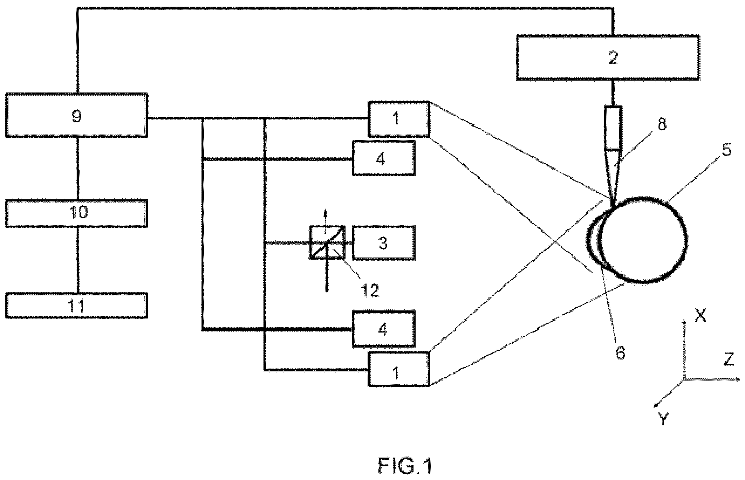

Although two second projectors (4) are depicted in Figure 1, the presence of a single second projector (4) projecting the slotted illumination pattern would suffice.

Hereinafter, to describe at least one embodiment of the device for obtaining mechanical, geometric, and dynamic measurements of optical surfaces, it will be assumed that the optical surface is the *in vivo* eye of a patient, the first optical region (5) is the sclera of the eye, and the second optical region (6) is the cornea of the eye.

Nevertheless, the optical surface may be an optical lens, an ophthalmic lens, a contact lens, an intraocular lens, or a self-adjusting lens.

According to a preferred embodiment of the invention, the spatially discrete stimulus generator (2) is a micro-air pulse generator configured for projecting a micro-air pulse on the surface of the sclera and/or cornea, preferably only on the surface of the sclera (8).

As an alternative to the micro-air pulse generator, another type of spatially discrete stimulus generator (2) which, (like in the case of the micro-air pulse generator), strikes an extremely small, spatially discrete area of the first optical region (5) (sclera), in a punctual instant, causing a controlled strain which propagates over the surface of the second optical region (6) (cornea), could be used.

As an example of an alternative to the micro-air pulse generator, a low-power laser source or an acoustic wave causing a controlled and ephemeral contraction of the surface of the eye in the sclera could be used, this strain propagating due to the contraction along the corneal surface.

The high-speed stereoscopic image generation system (1) can be achieved by means of a high-speed stereoscopic camera.

Likewise, as an alternative to the stereoscopic camera, a combination of two or more high-speed (not necessarily stereoscopic) cameras (1) could be used, which cameras together generate a stereoscopic image of the surface of the eye. Likewise, although less preferred, a high-speed (non-stereoscopic) camera can be used in combination with a set of mirrors or prism which provides a stereoscopic image.

In a preferred embodiment, the high-speed stereoscopic imaging system (1) may comprise at least two high-speed cameras.

Finally, it will be assumed that the spatially discrete stimulus generator (2) is a micro-air pulse generator configured for directing a micro-air pulse on the sclera of the eye.

This invention is characterized by the use of a projector (3) with infrared light illumination LED of different diameters for generating the random infrared illumination pattern (7) which is projected onto the entire corneal surface.

In one embodiment of the invention, the distribution of the random infrared illumination pattern (7) represents the point density which the cornea tolerates in a strain-free state and is calculated by tracing beams on the evaluation surface of the cornea.

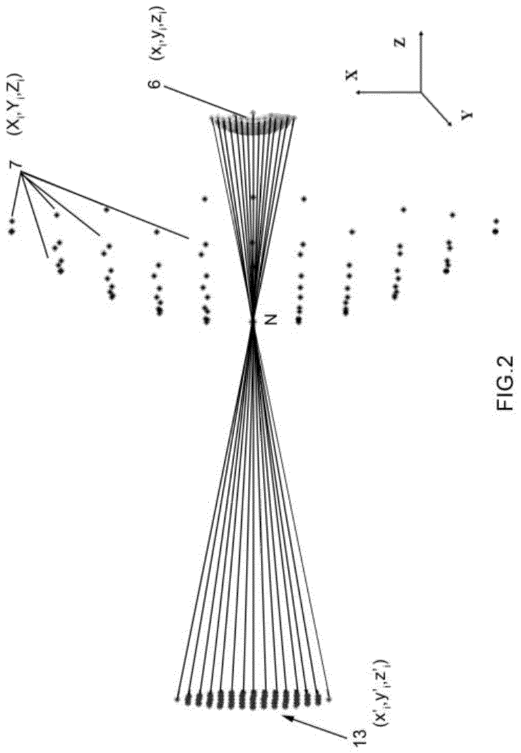

The random infrared illumination pattern (7) projected onto the entire surface of the second optical region (6) (cornea) by means of ray tracing can be schematically seen in Figure 2. In this figure, each light point of the random infrared illumination pattern (7) is projected towards the corneal surface with a given angle and a given position; these parameters are associated with the coordinates of the random infrared illumination pattern (7) by means of their origin and direction cosine of the propagation vectors of the light points of the random infrared illumination pattern (7). This therefore leads to obtaining the origin coordinate and the direction cosine of the propagation vectors for each point of the random infrared illumination pattern (7) (Xᵢ, Yᵢ, Zᵢ). The propagation vectors associated with the random infrared illumination pattern (7) strike the corneal surface in a specific position (xᵢ, yᵢ, zᵢ) for each light beam. On the corneal surface, each light beam is subject to reflection and then the direction of the direction cosine of the incident beams changes according to their angle of incidence with the normal to the corneal surface for each light point. The beams reflected by the corneal surface continue to propagate in a straight line towards the image plane (13) where at least one high-speed camera obtaining the point distribution x'ᵢ, y'ᵢ, z'ᵢ would be located. The point N where the reflected beams intersect with the optical axis is called a nodal point.

The distribution of points reflected (x'ᵢ, y'ᵢ, z'ᵢ) by the cornea and recorded by the high-speed stereoscopic imaging system (1), in the image plane (13), can be fitted to parametric models (sphere, conicoid, biconicoid, or Zernike polynomials), and relevant geometric information about the shape of the cornea such as, for example, the radius of curvature, asphericity, irregularity, aberrations, and topography, can thereby be obtained. The device of the invention can therefore be used as a dynamic corneal topography system.

The light output of the projectors (3, 4) preferably complies with the photobiological safety values set forth in the IEC/EN 62471 standard. The maximum illumination threshold will be set at at least one order of magnitude below the exposure limits described in ocular safety guidelines.

Each high-speed camera records the acquisition of the random infrared illumination pattern projected onto the entire corneal surface. The high-speed cameras also register the slotted (white light) illumination pattern projected on the central area of the cornea from the moment the micro-air pulse strikes the scleral region and during the propagation of the stimulus along the corneal surface. The superposition of the images obtained by each of the high-speed cameras thereby generates a final image with three-dimensional information measured in the entire extension of the cornea.

The separation and convergence of the planes of the high-speed cameras allow the stereoscopic (three-dimensional) capture of the image of the different illumination patterns that is reflected on the corneal surface.

To perform proper tracking of the coordinates of a point in space, the planes of the images obtained by means of at least one high-speed camera must capture images of the same point of the object (cornea-scleral region) in the same instant; therefore, the high-speed cameras are synchronized in real time for acquiring images in the same instant, and they are also synchronized both with the micro-air pulse focused on the scleral region and with the illumination patterns ((i) infrared illumination pattern (7) with random distribution over the entire corneal surface; and (ii) slotted illumination pattern on the central area of the cornea). For this synchronization to take place, the device object of the invention has a synchronization unit (9). The device object of the invention is therefore capable of capturing dynamic changes of the cornea in a controlled manner and with a high image acquisition speed. According to a possible embodiment, this acquisition speed can be 10,000 images per second, although it may be higher or lower than this value.

Once the high-speed cameras have been synchronized, the important thing is to know the rigid transformation which relates the position thereof relative to one another by knowing the position vector which goes, in the system, from one camera to another, and by knowing the angles (α, β, θ) of orientation of one camera with respect to the other camera.

According to a preferred embodiment, the device object of the invention also comprises an algorithm implemented in a processor (10); said algorithm is configured for calibrating the high-speed cameras, calculating the position of the high-speed cameras in space, and establishing the depth ratios between the high-speed cameras. Furthermore, the algorithm is configured for determining the position of the object (cornea or another surface or optical object) in space and the depth ratio of the object with respect to the high-speed cameras.

The processor (10) is configured (preferably by means of the mentioned algorithm) for generating a final image with three-dimensional information about the entire extension of the second optical region (6) using a superposition, by means of digital image correlation, of the images obtained by means of the high-speed stereoscopic imaging system (1).

The stereo triangulation of the image correlation can thereby be performed. Furthermore, the device object of the invention has a storage module (11) for storing images from the high-speed cameras, where said storage module (11) is connected to the processor (10)

The device object of the invention is equipped with an additional fixation channel (12) which allows controlling, at all times, the fixation of the patient, minimizing variabilities characteristic of the eye as much as possible.

The device object of the present invention can also be used as a dynamic topographer of the cornea (6) as a result of dynamic image acquisition in the initial moment without incidence of the air puff.

The method for obtaining mechanical, geometric, and dynamic measurements of optical surfaces object of the invention comprises the following steps:
- projecting a random infrared illumination pattern (7) onto the entire corneal surface;
- projecting a slotted white light pattern on the central area of the cornea;
- recording, by means of image acquisition by the high-speed stereoscopic system, the morphology of the sclera (limbus and blood vessels);
- acquiring at least one resting-state image (without incidence of the micro-air pulse) of the reflection of these illumination patterns on the cornea;
- applying a micro-air pulse in the scleral and/or corneal region;
- acquiring, by means of the stereoscopic system, high-speed images of the reflection of the projected illumination patterns on the cornea;
- removing the micro-air pulse;
- acquiring, by means of the stereoscopic system, high-speed images of the reflection of the projected illumination patterns on the cornea without incidence of the micro-air pulse; and
- detecting the process of recovery of the cornea to the initial state.

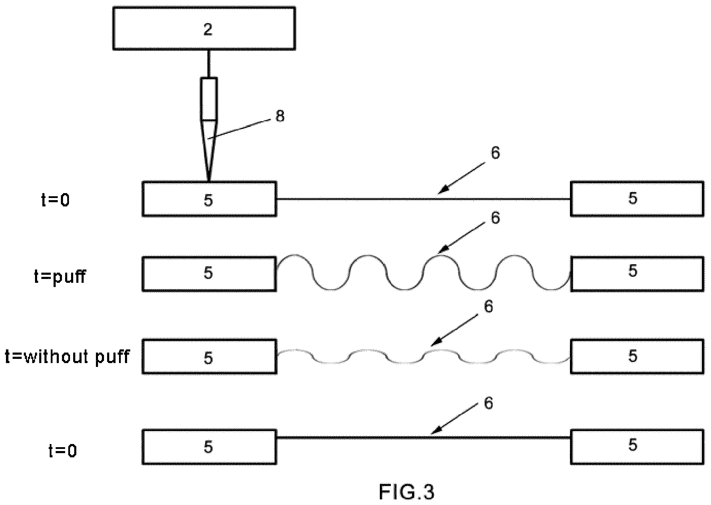

A diagram of the method can be seen in Figure 3, in which the second optical region (6) (cornea) and the first optical region (5) (scleral region) are schematically depicted in the resting state (initial instant, t=0), as well as during the application of the micro-air pulse on the scleral region (instant t=puff), and the modification of the position of the cornea and its subsequent recovery (t=without pulse) to the initial position (t=0) after the application of the micro-air pulse on the scleral and/or corneal region.

Once the images captured with the high-speed cameras are collected, they are preferably processed by means of the algorithm implemented in the processor (10) to detect, in each moment, the reflection of the random infrared illumination pattern (7) on the cornea, and to thereby quantify the dynamic response of the cornea to the micro-air pulse in a three-dimensional manner: from the initial position (without incidence of the micro-air pulse), to the position in each moment of the incidence of the micro-air pulse, until the stoppage of the micro-air pulse and the final recovery of the cornea.

As mentioned above, the spatially discrete stimulus (8) (e.g., the micro-air pulse) is applied in a punctual instant in time, and the duration thereof is preferably less than 1.5 seconds; more preferably, the spatially discrete stimulus (e.g., the micro-air pulse) is controlled so that the duration thereof is between 200 microseconds and 1 second.

Starting with an initial reference image of the cornea taken before projecting the micro-air pulse, the position of initial coordinates (xᵢ, yᵢ, zᵢ) corresponding to the random infrared illumination pattern (7) reflected on the cornea is detected.

Then, the test coordinates in each image (obtained by means of the high-speed cameras) are determined during the testing process (xi+ti, yi+ti, zi+ti) during the incidence of the micro-air pulse.

Finally, once the micro-air pulse is stopped, the final coordinates (x_{f}+t_{f}, y_{f}+t_{f}, z_{f}+t_{f}) are determined in the process of recovery to the initial state.

Each cornea has different recovery times. Typically, the recovery of the cornea to the initial state is recorded for a time period of two seconds. Nevertheless, this time can be regulated.

Therefore, by means of correlating the pixels between the different images obtained by means of the high-speed cameras, the differences between the positions of the coordinates can be calculated and a field of displacements which (given that the characteristics of the applied micro-air pulse are known) determine the mechanical properties of the cornea (by making a connection between the applied force, strain, recovery time) can be created.

To calculate the displacement, each image is uniformly divided into different subsets having a size of (2L+1)x(2L+1) pixels (where L represents the set of the region of analysis), and the displacement is calculated by non-linear variation, with the interpolation of intensity levels at the points between pixels being necessary.

Each subset is processed to calculate the mean value of the measurements or the mean values of the patterns of the region of analysis. For any one point P of the image, the corresponding subset is read and a search for the same subset is performed in the strained image. The displacement being calculated is therefore an average of the displacements of all the pixels of the subset. For the process of searching in the strained image, the degree of similarity between the reference subset and strained subset is evaluated. A correlation criterion is established for evaluating each of the subset of the strained image in the reference image. When the calculation of the correlation ends, it results in a displacement matrix for each subset of the initial or reference image.

As indicated, calculations are first performed in the initial position and the displacement is then calculated for all the pixels of the strained images using an optimal correlation path. The results are a displacement matrix for each subset into which the initial image was divided, said matrix allowing the precise determination of strains with a resolution in the order of microns. At the coordinates of the cornea (6) corresponding to the projection of the infrared LEDs, a series of ocular parameters with symmetry of revolution (pupil and white-white of the eye corresponding to the transition from the cornea (6) to the sclera) and/or particular parameters of each eye such as vessels or peculiarities present in the iris, are incorporated. Furthermore, the parameter corresponding to the slotted illumination pattern on the central area of the cornea (6) is added.

Finally, the proposed technique measures intraocular pressure (IOP) and integrates the correction of the intraocular pressure value based on the three-dimensional geometric and mechanical data of the cornea (6). IOP is the numerical intraocular pressure value in mmHg, determined by the ratio between the load applied in the cornea and the resistance of the cornea to the load. As mentioned in the background of the invention, given that the cornea is not a fully elastic and spherical structure having a single thickness all throughout, in order to obtain a real and precise IOP value there is a need to include a corrective parameter which is extracted from the three-dimensional mechanical parameters of the cornea. Furthermore, there is a possibility of integrating this data set in a finite element modeling program.

Based on the results, each of the displacements are obtained directly, or the strains indirectly, by applying computing means based on mechanical 3D DIC modeling, in which the stress-strain response and the viscoelastic and elastic response coefficients are analyzed.

Finally, with the three-dimensional (mechanical and geometric) parameters that are obtained, the real intraocular pressure value can be obtained as a result of the non-invasive mechanical stimulation of the cornea and the correction thereof with the three-dimensional mechanical data that is obtained.

The invention must not be limited to the particular embodiment described in this document. Those skilled in the art will be able to develop other embodiments in view of the description provided herein. Accordingly, the scope of the invention is defined by the following claims.

## Claims

1. A device for obtaining mechanical, geometric, and dynamic measurements of optical surfaces **characterized in that** it comprises:
- a high-speed stereoscopic imaging system (1);
- a spatially discrete stimulus generator (2) configured for directing a spatially discrete stimulus (8) to a first optical region (5) and to a second optical region (6);
- a first projector (3) configured for projecting a random infrared illumination pattern (7) onto the entire surface of the second optical region (6);
- a second projector (4) configured for projecting a slotted illumination pattern projected on the central area of the second optical region (6);
- a processor (10) configured for calibrating the high-speed stereoscopic imaging system (1), calculating the position of the high-speed stereoscopic imaging system (1) in space, establishing depth ratios in the high-speed stereoscopic imaging system (1), and determining the position of the second optical region (6) in space and the depth ratio of the second optical region (6) with respect to the high-speed stereoscopic imaging system (1);
wherein the high-speed stereoscopic imaging system (1) is configured for obtaining at least one image of the random infrared illumination pattern (7) projected onto the entire surface of the second optical region (6), as well as of the slotted illumination pattern projected onto the central area of the second optical region (6) without the application of the spatially discrete stimulus (8), and also for obtaining images of the random infrared illumination pattern (7) and of the slotted illumination pattern during and after the application of the spatially discrete stimulus (8) which strikes the first optical region (5) and the second optical region (6) and propagates over the surface of the second optical region (6); and
wherein the processor (10) is configured for generating a final image with three-dimensional information about the entire extension of the second optical region (6) using a superposition, by means of digital image correlation, of the images obtained by means of the high-speed stereoscopic imaging system (1).

2. The device for obtaining mechanical, geometric, and dynamic measurements of optical surfaces according to claim 1, **characterized in that**:
the first projector (3) comprises a plurality of infrared light LEDs and is configured for generating the random infrared illumination pattern (7); and/or
the second projector (4) comprises a white light source for generating the slotted illumination pattern.

3. The device for obtaining mechanical, geometric, and dynamic measurements of optical surfaces according to any of the preceding claims, **characterized in that** the first projector (3) is configured so that each beam of the random infrared illumination pattern (7) strikes the surface of the second optical region (6) and is reflected such that each beam reflected on the surface of the second optical region (6) is directed to an image plane (13) where the high-speed stereoscopic imaging system (1) obtaining a point distribution of the random infrared illumination pattern (7) is located.

4. The device for obtaining mechanical, geometric, and dynamic measurements of optical surfaces according to any of the preceding claims, **characterized in that** it comprises a synchronization unit (9) configured for synchronizing in real time the images obtained by means of the high-speed stereoscopic imaging system (1) with the isolated stimulus (8) focused on the first optical region (5) and on the second optical region (6), with the random infrared illumination pattern (7), and with the slotted illumination pattern.

5. The device for obtaining mechanical, geometric, and dynamic measurements of optical surfaces according to claim 1, **characterized in that** it comprises a storage module (11) for storing the images obtained by means of the high-speed stereoscopic imaging system (1), wherein said storage module (11) is connected to the processor (10).

6. The device for obtaining mechanical, geometric, and dynamic measurements of optical surfaces according to any of claims 2 to 5, **characterized in that** it comprises an additional fixation channel (12) configured for controlling a fixation of a patient.

7. The device for obtaining mechanical, geometric, and dynamic measurements of optical surfaces according to any of the preceding claims, **characterized in that** the high-speed stereoscopic imaging system (1) comprises a high-speed stereoscopic camera or at least two high-speed cameras.

8. The device for obtaining mechanical, geometric, and dynamic measurements of optical surfaces according to claim 7, **characterized in that** each light point of the random infrared illumination pattern (7) is projected towards the surface of the second optical region (6) with a given angle and a given position, which are associated with the coordinates of the random infrared illumination pattern (7) by means of their origin and direction cosine of the propagation vectors of the light points of the random infrared illumination pattern (7), and wherein the propagation vectors associated with the random infrared illumination pattern (7) strike the surface of the second optical region (6) in a specific position (xᵢ, yᵢ, zᵢ) for each light beam; wherein each light beam is subject to reflection on the surface of the second optical region (6), such that by means of said reflection it changes the direction of the direction cosine of the incident beams according to their angle of incidence with the normal to the surface of the second optical region (6) for each light point, such that the beams reflected by the surface of the second optical region (6) continue to propagate in a straight line towards the image plane (13) where at least one high-speed camera obtaining a point distribution (x'ᵢ, y'ᵢ, z'ᵢ) is located.

9. The device for obtaining mechanical, geometric, and dynamic measurements of optical surfaces according to any of the preceding claims, **characterized in that** the spatially discrete stimulus generator (2) comprises a micro-air pulse generator configured for directing a micro-air pulse to the first optical region (5) and/or to the second optical region (6).

10. A method for obtaining mechanical, geometric, and dynamic measurements of optical surfaces, **characterized in that** it comprises:
- projecting, by means of a first infrared light projector (3), a random infrared illumination pattern (7) onto the entire surface of a second optical region (6);
- projecting, by means of a second projector (4), a slotted illumination pattern on the central area of the second optical region (6);
- acquiring, by means of a high-speed stereoscopic imaging system (1), at least one resting-state image of the reflection of the illumination patterns on the second optical region (6);
- recording, by means of the high-speed stereoscopic imaging system (1), at least one resting-state image of the morphological patterns of a first optical region (5) and of the second optical region (6);
- applying, by means of a spatially discrete stimulus generator (2), a spatially discrete stimulus (8) focused on the first optical region (5) and on the second optical region (6);
- acquiring, by means of the high-speed stereoscopic imaging system (1), a plurality of high-speed images of the reflection on the second optical region (6) of the projected illumination patterns;
- again recording, by means of the high-speed stereoscopic imaging system (1), a plurality of high-speed images of the morphological patterns of the first optical region (5) and of the second optical region (6);
- removing the spatially discrete stimulus (8) that has been applied;
- acquiring, by means of the high-speed stereoscopic imaging system (1), a plurality of high-speed images of the reflection on the second optical region (6) of the projected illumination patterns without incidence of the spatially discrete stimulus (8);
- again recording, by means of the high-speed stereoscopic imaging system (1), a plurality of high-speed images of the morphological patterns of the first optical region (5) and of the second optical region (6); and
- measuring mechanical, geometric, and dynamic parameters of the second optical region (6) in the recovery thereof from the removal of the spatially discrete stimulus (8) to the state prior to the application of the spatially discrete stimulus (8).

11. The method for obtaining mechanical, geometric, and dynamic measurements of optical surfaces according to claim 10, **characterized in that** the first optical region (5) is the sclera of an eye and the second optical region (6) is the cornea of the eye.

12. The method for obtaining mechanical, geometric, and dynamic measurements of optical surfaces according to any of claims 10 to 11, **characterized in that**:
the first projector (3) comprises a plurality of infrared light LEDs and is configured for generating the random infrared illumination pattern (7); and/or
the second projector (4) comprises a white light source for generating a slotted illumination pattern.

13. The method for obtaining mechanical, geometric, and dynamic measurements of optical surfaces according to any of claims 10 to 12, **characterized in that** it comprises projecting the random infrared illumination pattern (7) such that each beam of the random infrared illumination pattern (7) strikes the surface of the second optical region (6) and is reflected such that each beam reflected on the surface of the second optical region (6) is directed to an image plane (13) where the high-speed stereoscopic imaging system (1) obtaining a point distribution of the random infrared illumination pattern (7) is located.

14. The method for obtaining mechanical, geometric, and dynamic measurements of optical surfaces according to any of claims 10 to 13, **characterized in that** the spatially discrete stimulus generator (2) comprises a micro-air pulse generator configured for directing a micro-air pulse to the first optical region (5) and/or to the second optical region (6).

15. The method for obtaining mechanical, geometric, and dynamic measurements of optical surfaces according to any of claims 10 to 14, **characterized in that** the duration of the spatially discrete stimulus (8) is less than 1.5 seconds, preferably between 200 microseconds and 1 second.

16. Use of:
- the device according to claim 1, wherein the first optical region (5) is the sclera of an eye and the second optical region (6) is the cornea of the eye; or
- the device according to any of claims 2 to 9;
wherein said use is selected from the group consisting of:
(a) use as a dynamic corneal topography system, by means of acquiring images without incidence of the spatially discrete stimulus (8);
(b) use for the three-dimensional analysis of the geometric, mechanical, and dynamic properties of the cornea;
(c) use for the analysis of intraocular pressure;
(d) use for the evaluation, classification, and follow-up of people without ocular pathologies, for determining the threshold value indicative of the onset of at least the following ocular pathologies:
i. pathologies weakening the corneal structure such as corneal ectasia and keratoconus;
ii. glaucoma; and
iii. dry eye;
thereby improving predictability in the diagnosis of said ocular pathologies and enabling the determination of a suitable treatment for said pathologies.

17. Use of the device according to any of claims 1 to 9 for the three-dimensional analysis of the shape and mechanics of optical and electro-optical elements and devices, such as ophthalmic lenses, contact lenses, intraocular lenses, self-adjusting lenses.

## Patentansprüche

1. Vorrichtung zum Erhalten mechanischer, geometrischer und dynamischer Messungen von optischen Oberflächen, **dadurch gekennzeichnet, dass** sie aufweist:
- ein stereoskopisches Hochgeschwindigkeits-Abbildungssystem (1);
- einen räumlich diskreten Stimulusgenerator (2), derart konfiguriert, um einen räumlich diskreten Stimulus (8) auf ein erstes optisches Gebiet (5) und auf ein zweites optisches Gebiet (6) zu richten;
- einen ersten Projektor (3), der derart konfiguriert ist, dass er ein zufälliges Infrarot-Beleuchtungsmuster (7) auf die gesamte Oberfläche des zweiten optischen Gebiets (6) projiziert;
- einen zweiten Projektor (4), der derart konfiguriert ist, dass er ein geschlitztes Beleuchtungsmuster projiziert, das auf die zentrale Fläche des zweiten optischen Gebiets (6) projiziert wird;
- einen Prozessor (10), der derart konfiguriert ist, dass er das stereoskopische Hochgeschwindigkeits-Abbildungssystem (1) kalibriert, die Position des stereoskopischen Hochgeschwindigkeits-Abbildungssystems (1) im Raum berechnet, Tiefenverhältnisse in dem stereoskopischen Hochgeschwindigkeits-Abbildungssystem (1) festlegt und die Position des zweiten optischen Gebiets (6) im Raum und das Tiefenverhältnis des zweiten optischen Gebiets (6) in Bezug auf das stereoskopische Hochgeschwindigkeits-Abbildungssystem (1) bestimmt;
wobei das stereoskopische Hochgeschwindigkeits-Abbildungssystem (1) derart konfiguriert ist, dass es mindestens ein Bild des zufälligen Infrarot-Beleuchtungsmusters (7), das auf die gesamte Fläche des zweiten optischen Gebiets (6) projiziert wird, sowie des geschlitzten Beleuchtungsmusters, das auf die zentrale Fläche des zweiten optischen Gebiets (6) projiziert wird, ohne die Anwendung des räumlich diskreten Stimulus (8) erhält, und auch dass es Bilder des zufälligen Infrarot-Beleuchtungsmusters (7) und des geschlitzten Beleuchtungsmusters während und nach der Anwendung des räumlich diskreten Stimulus (8), der auf das erste optische Gebiet (5) und das zweite optische Gebiet (6) trifft und sich über die Oberfläche des zweiten optischen Gebiets (6) ausbreitet, erhält; und
wobei der Prozessor (10) derart konfiguriert ist, dass er ein Endbild mit dreidimensionaler Information über die gesamte Ausdehnung des zweiten optischen Gebiets (6) erzeugt, indem er mittels digitaler Bildkorrelation eine Überlagerung der mittels des stereoskopischen Hochgeschwindigkeits-Abbildungssystems (1) erhaltenen Bilder verwendet.

2. Vorrichtung zum Erhalten mechanischer, geometrischer und dynamischer Messungen von optischen Oberflächen nach Anspruch 1, **dadurch gekennzeichnet, dass**
der erste Projektor (3) eine Vielzahl von Infrarot-LEDs aufweist und derart konfiguriert ist, dass er das zufällige Infrarot-Beleuchtungsmuster (7) erzeugt; und/oder
der zweite Projektor (4) eine Weißlichtquelle zur Erzeugung des geschlitzten Beleuchtungsmusters aufweist.

3. Vorrichtung zum Erhalten mechanischer, geometrischer und dynamischer Messungen von optischen Oberflächen nach einem der vorhergehenden Ansprüche, **dadurch gekennzeichnet, dass** der erste Projektor (3) derart konfiguriert ist, dass jeder Strahl des zufälligen Infrarot-Beleuchtungsmusters (7) auf die Oberfläche des zweiten optischen Gebiets (6) trifft und so reflektiert wird, dass jeder an der Oberfläche des zweiten optischen Gebiets (6) reflektierte Strahl auf eine Bildebene (13) gerichtet ist, in der sich das stereoskopische Hochgeschwindigkeits-Abbildungssystem (1) befindet, das eine Punktverteilung des zufälligen Infrarot-Beleuchtungsmusters (7) erhält.

4. Vorrichtung zum Erhalten mechanischer, geometrischer und dynamischer Messungen von optischen Oberflächen nach einem der vorhergehenden Ansprüche, **dadurch gekennzeichnet, dass** sie eine Synchronisationseinheit (9) aufweist, die derart konfiguriert ist, dass sie in Echtzeit die mittels des stereoskopischen Hochgeschwindigkeits-Abbildungssystems (1) erhaltenen Bilder mit dem isolierten Stimulus (8), der auf das erste optische Gebiet (5) und das zweite optische Gebiet (6) fokussiert ist, mit dem zufälligen Infrarot-Beleuchtungsmuster (7) und mit dem geschlitzten Beleuchtungsmuster synchronisiert.

5. Vorrichtung zum Erhalten mechanischer, geometrischer und dynamischer Messungen von optischen Oberflächen gemäß Anspruch 1, **dadurch gekennzeichnet, dass** sie ein Speichermodul (11) zum Speichern der mittels des stereoskopischen Hochgeschwindigkeits-Abbildungssystems (1) erhaltenen Bilder aufweist, wobei das Speichermodul (11) mit dem Prozessor (10) verbunden ist.

6. Vorrichtung zum Erhalten mechanischer, geometrischer und dynamischer Messungen von optischen Oberflächen nach einem der Ansprüche 2 bis 5, **dadurch gekennzeichnet, dass** sie einen zusätzlichen Fixierungskanal (12) aufweist, der derart konfiguriert ist, dass er die Fixierung eines Patienten steuert.

7. Vorrichtung zum Erhalten mechanischer, geometrischer und dynamischer Messungen von optischen Oberflächen nach einem der vorhergehenden Ansprüche, **dadurch gekennzeichnet, dass** das stereoskopische Hochgeschwindigkeits-Abbildungssystem (1) eine stereoskopische Hochgeschwindigkeitskamera oder mindestens zwei Hochgeschwindigkeitskameras aufweist.

8. Vorrichtung zum Erhalten mechanischer, geometrischer und dynamischer Messungen von optischen Oberflächen nach Anspruch 7, **dadurch gekennzeichnet, dass** jeder Lichtpunkt des zufälligen Infrarot-Beleuchtungsmusters (7) auf die Oberfläche des zweiten optischen Gebiets (6) projiziert wird mit einem bestimmten Winkel und einer bestimmten Position, die den Koordinaten des zufälligen Infrarot-Beleuchtungsmusters (7) mittels ihres Ursprungs und des Richtungskosinus der Ausbreitungsvektoren der Lichtpunkte des zufälligen Infrarot-Beleuchtungsmusters (7) zugeordnet sind, und wobei die dem zufälligen Infrarot-Beleuchtungsmuster (7) zugeordneten Ausbreitungsvektoren auf die Oberfläche des zweiten optischen Gebiets (6) in einer bestimmten Position (xᵢ, yᵢ, zᵢ) für jeden Lichtstrahl treffen; wobei jeder Lichtstrahl einer Reflexion an der Oberfläche des zweiten optischen Gebiets (6) unterliegt, so dass es durch diese Reflexion die Richtung des Richtungskosinus der einfallenden Lichtstrahlen entsprechend ihrem Einfallswinkel mit der Senkrechten auf die Oberfläche des zweiten optischen Gebiets (6) für jeden Lichtpunkt ändert, so dass sich die von der Oberfläche des zweiten optischen Gebiets (6) reflektierten Strahlen weiterhin in einer geraden Linie in Richtung der Bildebene (13) ausbreiten, in der sich mindestens eine Hochgeschwindigkeitskamera befindet, die eine Punktverteilung (x'ᵢ, y' ᵢ, z'ᵢ) erhält.

9. Vorrichtung zum Erhalten mechanischer, geometrischer und dynamischer Messungen von optischen Oberflächen nach einem der vorhergehenden Ansprüche, **dadurch gekennzeichnet, dass** der räumlich diskrete Stimulusgenerator (2) einen derart konfigurierten Mikroluftimpulsgenerator aufweist, dass er einen Mikroluftimpuls auf das erste optische Gebiet (5) und/oder das zweite optische Gebiet (6) richtet.

10. Verfahren zum Erhalten mechanischer, geometrischer und dynamischer Messungen von optischen Oberflächen, **dadurch gekennzeichnet, dass** es Folgendes umfasst:
- Projizieren, mittels eines ersten Infrarot-Lichtprojektors (3), eines zufälligen Infrarot-Beleuchtungsmusters (7) auf die gesamte Oberfläche eines zweiten optischen Gebiets (6);
- Projizieren, mittels eines zweiten Projektors (4), eines geschlitzten Beleuchtungsmusters auf die zentrale Fläche des zweiten optischen Gebiets (6);
- Erfassen, mittels eines stereoskopischen Hochgeschwindigkeits-Abbildungssystems (1), mindestens eines Ruhezustandsbildes der Reflexion der Beleuchtungsmuster auf dem zweiten optischen Gebiet (6);
- Aufnehmen, mittels des stereoskopischen Hochgeschwindigkeits-Abbildungssystems (1), mindestens eines Ruhezustandsbildes der morphologischen Muster eines ersten optischen Gebietes (5) und des zweiten optischen Gebietes (6);
- Anlegen, mittels eines räumlich diskreten Stimulusgenerators (2), eines räumlich diskreten Stimulus (8), der auf das erste optische Gebiet (5) und auf das zweite optische Gebiet (6) fokussiert ist;
- Erfassen, mittels des stereoskopischen Hochgeschwindigkeits-Abbildungssystems (1), einer Vielzahl von Hochgeschwindigkeitsbildern der Reflexion, auf dem zweiten optischen Gebiet (6), der projizierten Beleuchtungsmuster;
- wiederum Aufnehmen, mittels des stereoskopischen Hochgeschwindigkeits-Abbildungssystems (1), einer Vielzahl von Hochgeschwindigkeitsbildern der morphologischen Muster des ersten optischen Gebiets (5) und des zweiten optischen Gebiets (6);
- Entfernen des angelegten räumlich diskreten Stimulus (8);
- Erfassen, mittels des stereoskopischen Hochgeschwindigkeits-Abbildungssystems (1), einer Vielzahl von Hochgeschwindigkeitsbildern der Reflexion, auf dem zweiten optischen Gebiet (6), der projizierten Beleuchtungsmuster ohne Einfall des räumlich diskreten Stimulus (8);
- wiederum Aufnehmen, mittels des stereoskopischen Hochgeschwindigkeits-Abbildungssystems (1), einer Vielzahl von Hochgeschwindigkeitsbildern der morphologischen Muster des ersten optischen Gebietes (5) und des zweiten optischen Gebiets (6); und
- Messen mechanischer, geometrischer und dynamischer Parameter des zweiten optischen Gebiets (6) bei der Rückführung desselben von der Entfernung des räumlich diskreten Stimulus (8) in den Zustand vor der Anwendung des räumlich diskreten Stimulus (8).

11. Verfahren zum Erhalten mechanischer, geometrischer und dynamischer Messungen von optischen Oberflächen nach Anspruch 10, **dadurch gekennzeichnet, dass** das erste optische Gebiet (5) die Sklera eines Auges ist und das zweite optische Gebiet (6) die Hornhaut des Auges ist.

12. Verfahren zum Erhalten mechanischer, geometrischer und dynamischer Messungen von optischen Oberflächen nach einem der Ansprüche 10 bis 11, **dadurch gekennzeichnet, dass**
der erste Projektor (3) eine Vielzahl von Infrarot-LEDs aufweist und derart konfiguriert ist, dass er das zufällige Infrarot-Beleuchtungsmuster (7) erzeugt; und/oder
der zweite Projektor (4) eine Weißlichtquelle zum Erzeugen eines geschlitzten Beleuchtungsmusters aufweist.

13. Verfahren zum Erhalten mechanischer, geometrischer und dynamischer Messungen von optischen Oberflächen nach einem der Ansprüche 10 bis 12, **dadurch gekennzeichnet, dass** es umfasst, das zufällige Infrarot-Beleuchtungsmuster (7) so zu projizieren, dass jeder Strahl des zufälligen Infrarot-Beleuchtungsmusters (7) auf die Oberfläche des zweiten optischen Gebiets (6) auftrifft und so reflektiert wird, dass jeder an der Oberfläche des zweiten optischen Gebiets (6) reflektierte Strahl auf eine Bildebene (13) gerichtet wird, in der sich das stereoskopische Hochgeschwindigkeits-Abbildungssystem (1) befindet, das eine Punktverteilung des zufälligen Infrarot-Beleuchtungsmusters (7) erhält.

14. Verfahren zum Erhalten mechanischer, geometrischer und dynamischer Messungen von optischen Oberflächen nach einem der Ansprüche 10 bis 13, **dadurch gekennzeichnet, dass** der räumlich diskrete Stimulusgenerator (2) einen derart konfigurierten Mikroluftimpulsgenerator aufweist, dass er einen Mikroluftimpuls auf das erste optische Gebiet (5) und/oder auf das zweite optische Gebiet (6) richtet.

15. Verfahren zum Erhalten mechanischer, geometrischer und dynamischer Messungen von optischen Oberflächen nach einem der Ansprüche 10 bis 14, **dadurch gekennzeichnet, dass** die Dauer des räumlich diskreten Stimulus (8) weniger als 1,5 Sekunden, vorzugsweise zwischen 200 Mikrosekunden und 1 Sekunde, beträgt.

16. Verwendung von:
- der Vorrichtung nach Anspruch 1, wobei das erste optische Gebiet (5) die Sklera eines Auges ist und das zweite optische Gebiet (6) die Hornhaut des Auges ist; oder
- der Vorrichtung nach einem der Ansprüche 2 bis 9;
wobei die Verwendung ausgewählt ist aus der Gruppe bestehend aus:
(a) Verwendung als dynamisches Hornhauttopographiesystem, indem Bilder ohne Einfall des räumlich diskreten Stimulus (8) aufgenommen werden;
(b) Verwendung für die dreidimensionale Analyse der geometrischen, mechanischen und dynamischen Eigenschaften der Hornhaut;
(c) Verwendung für die Analyse des Augeninnendrucks;
(d) Verwendung für die Bewertung, Klassifizierung und Nachverfolgung von Personen ohne Augenkrankheiten zur Bestimmung des Schwellenwerts, der auf das Auftreten mindestens der folgenden Augenkrankheiten hinweist:
i. Pathologien, die den Aufbau der Hornhaut schwächen, wie z. B. Hornhautektasie und Keratokonus;
ii. Glaukom; und
iii. trockenes Auge;
wodurch die Vorhersagbarkeit bei der Diagnose der genannten Augenkrankheiten verbessert und die Bestimmung einer geeigneten Behandlung für die genannten Krankheiten ermöglicht wird.

17. Verwendung der Vorrichtung nach einem der Ansprüche 1 bis 9 zur dreidimensionalen Analyse der Form und der Mechanik optischer und elektrooptischer Elemente und Vorrichtungen, wie ophthalmische Linsen, Kontaktlinsen, Intraokularlinsen, selbstanpassende Linsen.

## Revendications

1. Un dispositif d'obtention de mesures mécaniques, géométriques et dynamiques de surfaces optiques **caractérisé en ce qu'**il comprend:
- un système d'imagerie stéréoscopique à grande vitesse (1) ;
- un générateur de stimulus spatialement distincts (2) configuré pour diriger un stimulus spatialement distinct (8) vers une première zone optique (5) et vers une deuxième zone optique (6);
- un premier projecteur (3) configuré pour projeter un motif d'illumination infrarouge aléatoire (7) sur toute la surface de la deuxième zone optique (6);
- un deuxième projecteur (4) configuré pour projeter un motif d'éclairement à fentes projeté sur la zone centrale de la deuxième zone optique (6);
- un processeur (10) configuré pour calibrer le système d'imagerie stéréoscopique à grande vitesse (1), calculer la position du système d'imagerie stéréoscopique à grande vitesse (1) dans l'espace, établir des rapports de profondeur dans le système d'imagerie stéréoscopique à grande vitesse (1), et déterminer la position de la deuxième zone optique (6) dans l'espace et le rapport de profondeur de la deuxième zone optique (6) par rapport au système d'imagerie stéréoscopique à grande vitesse (1);
le système d'imagerie stéréoscopique à grande vitesse (1) étant configuré pour obtenir au moins une image du motif d'éclairement infrarouge aléatoire (7) projeté sur toute la surface de la deuxième zone optique (6), ainsi que du motif d'éclairement à fentes projeté sur la zone centrale de la deuxième zone optique (6) sans l'application du stimulus spatialement distinct (8), et également pour obtenir des images du motif d'éclairement infrarouge aléatoire (7) et du motif d'éclairement à fentes pendant et après l'application du stimulus spatialement distinct (8) qui frappe la première zone optique (5) et la deuxième zone optique (6) et se propage sur la surface de la deuxième zone optique (6); et le processeur (10) étant configuré pour générer une image finale avec des informations tridimensionnelles sur toute l'extension de la deuxième zone optique (6) en utilisant une superposition, au moyen d'une corrélation d'images numériques, des images obtenues au moyen du système d'imagerie stéréoscopique à grande vitesse (1).

2. Le dispositif d'obtention de mesures mécaniques, géométriques et dynamiques de surfaces optiques selon la revendication 1, **caractérisé en ce que**:
le premier projecteur (3) comprend une pluralité de LED à lumière infrarouge et est configuré pour générer le motif d'éclairement infrarouge aléatoire (7); et/ou
le deuxième projecteur (4) comprend une source de lumière blanche pour générer le motif d'éclairement à fentes.

3. Le dispositif d'obtention de mesures mécaniques, géométriques et dynamiques de surfaces optiques selon l'une quelconque des revendications précédentes, **caractérisé en ce que** le premier projecteur (3) est configuré de façon que chaque faisceau du motif d'éclairement infrarouge aléatoire (7) frappe la surface de la deuxième zone optique (6) et est réfléchi de telle sorte que chaque faisceau réfléchi sur la surface de la deuxième zone optique (6) est dirigé vers un plan image (13) au niveau duquel est situé le système d'imagerie stéréoscopique à grande vitesse (1) obtenant une distribution ponctuelle du motif d'éclairement infrarouge aléatoire (7).

4. Le dispositif d'obtention de mesures mécaniques, géométriques et dynamiques de surfaces optiques selon l'une quelconque des revendications précédentes, **caractérisé en ce qu'**il comprend une unité de synchronisation (9) configurée pour synchroniser en temps réel les images obtenues au moyen du système d'imagerie stéréoscopique à grande vitesse (1) avec le stimulus isolé (8) focalisé sur la première zone optique (5) et sur la deuxième zone optique (6), avec le motif d'éclairement infrarouge aléatoire (7) et avec le motif d'éclairement à fentes.

5. Le dispositif d'obtention de mesures mécaniques, géométriques et dynamiques de surfaces optiques selon la revendication 1, **caractérisé en ce qu'**il comprend un module de stockage (11) pour stocker les images obtenues au moyen du système d'imagerie stéréoscopique à grande vitesse (1), ledit module de stockage (11) étant connecté au processeur (10) .

6. Le dispositif d'obtention de mesures mécaniques, géométriques et dynamiques de surfaces optiques selon l'une quelconque des revendications 2 à 5, **caractérisé en ce qu'**il comprend un canal de fixation supplémentaire (12) configuré pour commander une fixation d'un patient.

7. Le dispositif d'obtention de mesures mécaniques, géométriques et dynamiques de surfaces optiques selon l'une quelconque des revendications précédentes, **caractérisé en ce que** le système d'imagerie stéréoscopique à grande vitesse (1) comprend un appareil de prise de vues stéréoscopique à grande vitesse ou au moins deux appareils de prise de vues à grande vitesse.

8. Le dispositif d'obtention de mesures mécaniques, géométriques et dynamiques de surfaces optiques selon la revendication 7, **caractérisé en ce que** chaque point lumineux du motif d'éclairement infrarouge aléatoire (7) est projeté vers la surface de la deuxième zone optique (6) avec un angle donné et une position donnée, qui sont associées aux coordonnées du motif d'éclairement infrarouge aléatoire (7) au moyen de leur origine et cosinus de direction des vecteurs de propagation des points lumineux du motif d'éclairement infrarouge aléatoire (7), et dans lequel les vecteurs de propagation associés au motif d'éclairement infrarouge aléatoire (7) frappent la surface de la deuxième zone optique (6) dans une position spécifique (xᵢ, yᵢ, zᵢ) pour chaque faisceau lumineux; chaque faisceau lumineux est soumis à une réflexion sur la surface de la deuxième zone optique (6), de telle sorte qu'au moyen de ladite réflexion il change la direction du cosinus de direction des faisceaux incidents en fonction de leur angle d'incidence avec la normale à la surface de la deuxième zone optique (6) pour chaque point lumineux, de telle sorte que les faisceaux réfléchis par la surface de la deuxième zone optique (6) continuent à se propager en ligne droite vers le plan image (13) au niveau duquel est situé au moins un appareil de prise de vues à grande vitesse obtenant une distribution de points (x'ᵢ, y'ᵢ, z'ᵢ).

9. Le dispositif d'obtention de mesures mécaniques, géométriques et dynamiques de surfaces optiques selon l'une quelconque des revendications précédentes, **caractérisé en ce que** le générateur de stimulus spatialement distincts (2) comprend un générateur d'impulsions micro-air configuré pour diriger une impulsion micro-air à la première zone optique (5) et/ou à la deuxième zone optique (6).

10. Un procédé d'obtention de mesures mécaniques, géométriques et dynamiques de surfaces optiques, **caractérisé en ce qu'**il comprend:
- le fait de projeter, au moyen d'un premier projecteur de lumière infrarouge (3), un motif d'éclairement infrarouge aléatoire (7) sur toute la surface d'une deuxième zone optique (6);
- le fait de projeter, au moyen d'un deuxième projecteur (4), un motif d'éclairement à fentes sur la zone centrale de la deuxième zone optique (6);
- le fait d'acquérir, au moyen d'un système d'imagerie stéréoscopique à grande vitesse (1), au moins une image en état de repos de la réflexion des motifs d'éclairement sur la deuxième zone optique (6);
- le fait d'enregistrer, au moyen du système d'imagerie stéréoscopique à grande vitesse (1), au moins une image en état de repos des motifs morphologiques d'une première zone optique (5) et de la deuxième zone optique (6);
- le fait d'appliquer, au moyen d'un générateur de stimulus spatialement distincts (2), un stimulus spatialement distinct (8) focalisé sur la première zone optique (5) et sur la deuxième zone optique (6);
- le fait d'acquérir, au moyen du système d'imagerie stéréoscopique à grande vitesse (1), une pluralité d'images à grande vitesse de la réflexion sur la deuxième zone optique (6) des motifs d'éclairement projetés;
- le fait d'enregistrer à nouveau, au moyen du système d'imagerie stéréoscopique à grande vitesse (1), une pluralité d'images à grande vitesse des motifs morphologiques de la première zone optique (5) et de la deuxième zone optique (6);
- le fait de supprimer le stimulus spatialement distinct (8) qui a été appliqué;
- le fait d'acquérir, au moyen du système d'imagerie stéréoscopique à grande vitesse (1), une pluralité d'images à grande vitesse de la réflexion sur la deuxième zone optique (6) des motifs d'éclairement projetés sans incidence du stimulus spatialement distinct (8);
- le fait d'enregistrer à nouveau, au moyen du système d'imagerie stéréoscopique à grande vitesse (1), une pluralité d'images à grande vitesse des motifs morphologiques de la première zone optique (5) et de la deuxième zone optique (6); et
- le fait de mesurer des paramètres mécaniques, géométriques et dynamiques de la deuxième zone optique (6) dans la récupération de celle-ci depuis la suppression du stimulus spatialement distinct (8) jusqu'à l'état antérieur à l'application du stimulus spatialement distinct (8).

11. Le procédé d'obtention de mesures mécaniques, géométriques et dynamiques de surfaces optiques selon la revendication 10, **caractérisé en ce que** la première zone optique (5) est la sclérotique d'un œil et la deuxième zone optique (6) est la cornée du œil.

12. Le procédé d'obtention de mesures mécaniques, géométriques et dynamiques de surfaces optiques selon l'une quelconque des revendications 10 à 11, **caractérisé en ce que**:
le premier projecteur (3) comprend une pluralité de LED à lumière infrarouge et est configuré pour générer le motif d'éclairement infrarouge aléatoire (7); et/ou
le deuxième projecteur (4) comprend une source de lumière blanche pour générer un motif d'éclairement à fentes.

13. Le procédé d'obtention de mesures mécaniques, géométriques et dynamiques de surfaces optiques selon l'une quelconque des revendications 10 à 12, **caractérisé en ce qu'**il comprend le fait de projeter le motif d'éclairement infrarouge aléatoire (7) de telle sorte que chaque faisceau du motif d'éclairement infrarouge aléatoire (7) frappe la surface de la deuxième zone optique (6) et est réfléchi de telle sorte que chaque faisceau réfléchi sur la surface de la deuxième zone optique (6) est dirigé vers un plan image (13) au niveau duquel est situé le système d'imagerie stéréoscopique à grande vitesse (1) obtenant une distribution ponctuelle du motif d'éclairement infrarouge aléatoire (7).

14. Le procédé d'obtention de mesures mécaniques, géométriques et dynamiques de surfaces optiques selon l'une quelconque des revendications 10 à 13, **caractérisé en ce que** le générateur de stimulus spatialement distincts (2) comprend un générateur d'impulsions micro-air configuré pour diriger une impulsion micro-air vers la première zone optique (5) et/ou vers la deuxième zone optique (6).

15. Le procédé d'obtention de mesures mécaniques, géométriques et dynamiques de surfaces optiques selon l'une quelconque des revendications 10 à 14, **caractérisé en ce que** la durée du stimulus spatialement distinct (8) est inférieure à 1,5 seconde, de préférence comprise entre 200 microsecondes et 1 seconde.

16. Utilisation:
- du dispositif selon la revendication 1, dans laquelle la première zone optique (5) est la sclérotique d'un œil et la deuxième zone optique (6) est la cornée de l'œil; ou
- du dispositif selon l'une quelconque des revendications 2 à 9;
ladite utilisation étant choisie dans le groupe constitué par:
(a) une utilisation en tant que système dynamique de topographie cornéenne, au moyen de l'acquisition d'images sans incidence du stimulus spatialement distinct (8);
(b) une utilisation pour l'analyse tridimensionnelle des propriétés géométriques, mécaniques et dynamiques de la cornée;
(c) une utilisation pour l'analyse de la pression intraoculaire;
(d) une utilisation pour l'évaluation, la classification et le suivi de personnes sans pathologies oculaires, pour la détermination de la valeur seuil indicative de l'apparition d'au moins les pathologies oculaires suivantes:
i. les pathologies fragilisant la structure cornéenne telles que l'ectasie cornéenne et le kératocône;
ii. le glaucome; et
iii. la sécheresse oculaire;
améliorant ainsi la prévisibilité dans le diagnostic desdites pathologies oculaires et permettant la détermination d'un traitement approprié pour lesdites pathologies.

17. Utilisation du dispositif selon l'une quelconque des revendications 1 à 9 pour l'analyse tridimensionnelle de la forme et de la mécanique d'éléments et dispositifs optiques et électro-optiques, tels que lentilles ophtalmiques, lentilles de contact, lentilles intraoculaires, lentilles auto-ajustables.
